# EUROPÄISCHE PATENTSCHRIFT

(11) **EP 4 703 346 B1**
(45) Veröffentlichungstag und Bekanntmachung des Hinweises auf die Patenterteilung: **25.11.2026**
(21) Anmeldenummer: 24198035.8
(22) Anmeldetag: 03.09.2024
(51) Int. Cl.: C08K 5/00, C08K 5/101, C08K 5/12, C08K 5/151, C07C 69/608, C07C 69/75

(54) **WEICHMACHERZUSAMMENSETZUNG ENTHALTEND TRIMETHYLESTER DER 1,2,4-CYCLOHEXANTRIPROPIONSÄURE UND 1,2-DIALKYLCYCLOHEXANDICARBONSÄUREESTER**
PLASTICIZER COMPOSITION COMPRISING TRIMETHYLESTER OF 1,2,4-CYCLOHEXANETRIPROPIONIC ACID AND 1,2-DIALKYLCYCLOHEXANEDICARBOXYLIC ACID ESTER
COMPOSITION PLASTIFIANTE CONTENANT DU TRIMÉTHYLESTER DE L'ACIDE 1,2,4-CYCLOHEXANETRIPROPIONIQUE ET DE L'ESTER 1,2-DIALKYLCYCLOHEXANE DICARBOXYLIQUE

(43) Veröffentlichungstag der Anmeldung: 04.03.2026
(73) Patentinhaber: Evonik Oxeno GmbH & Co. KG, 45772 Marl (DE)
(72) Erfinder: GRASS, Michael, 45721 Haltern am See (DE); RUSEK, Monika, 45149 Essen (DE); VAN EICKELS, Michael, 45657 Recklinghausen (DE)
(74) Vertreter: Evonik Patent Association

(56) Entgegenhaltungen:
- EP-A1- 3 838 886
- EP-A1- 3 838 888
- WO-A1-2020/246743
- WO-A2-2009/085453

## Beschreibung

Gegenstand der Erfindung ist eine Weichmacherzusammensetzung enthaltend eine Verbindung der Formel (1) 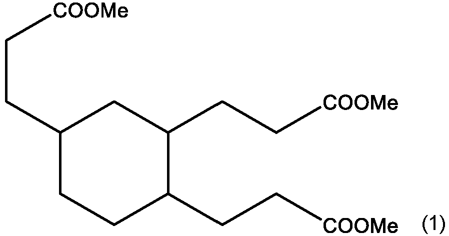 und mindestens einen 1,2-Dialkylcyclohexandicarbonsäureester, wobei die beiden Alkylgruppen im 1,2-Dialkylcyclohexandicarbonsäureester jeweils 8 oder 9 Kohlenstoffatome aufweisen.

Die erfindungsgemäßen Verbindungen nach der Formel (1) sind Trimethylester der 1,2,4-Cyclohexantripropionsäure. Trimethylester der 1,2,4-Cyclohexantripropionsäure sind grundsätzlich bekannt und beispielsweise in der EP 3 838 886 A1 beschrieben worden. Dort wurde auch erwähnt, dass diese Ester der 1,2,4-Cyclohexantripropionsäure als Weichmacher eingesetzt werden können. Der Trimethylester wurde in diesem Zusammenhang als ein Produkt mit niedriger Geliertemperatur beschrieben.

In der EP 3 838 888 A1 werden Triester durch Umesterung der Verbindung (1) hergestellt und als Weichmacher eingesetzt.

Die WO 2020/246743 A1 stellt eine Weichmacherzusammensetzung aus Tri(2-ethylhexyl)cyclohexane-1,2,4-tricarboxylate (TEHCH), einem epoxidierten Öl und einem Cyclohexandicarboxylate bereit.

In der WO2009/085453 A2 wird eine Zusammensetzung aus schnell und langsam schmelzender Weichmacher beschrieben. Als weiterer Weichmacher werden 1,2,4-Triester von Cyclohexantrisäure vorgeschlagen.

Diese Ester zeigen beim Einsatz als Weichmacher in Kunststoffen nicht immer nur positive Eigenschaften. Würde man den Trimethylester nämlich als einzigen Weichmacher, beispielsweise in einer PVC-basierten Plastisol-Rezeptur einsetzen, würde das Plastisol eine recht hohe Viskosität aufweisen. Außerdem würde bei der Verarbeitung des Plastisols ein nicht unerheblicher Anteil des Trimethylesters verdampfen und somit nicht mehr im Endprodukt verbleiben. Es macht daher Sinn die erwähnten Trimethylester in Mischungen mit anderen Weichmachern einzusetzen.

Der vorliegenden Erfindung lag nun die Aufgabe zugrunde, eine Weichmacherzusammensetzung bereitzustellen, die den Trimethylester der 1,2,4-Cyclohexantripropionsäure enthält, aber bessere anwendungstechnische Eigenschaften aufweist als Weichmacherzusammensetzungen mit anderen Schnellgelierern.

Diese Aufgabe wird gelöst durch die Weichmacherzusammensetzung nach Anspruch 1. Gegenstand der Erfindung ist demnach eine Weichmacherzusammensetzung enthaltend eine Verbindung der Formel (1) 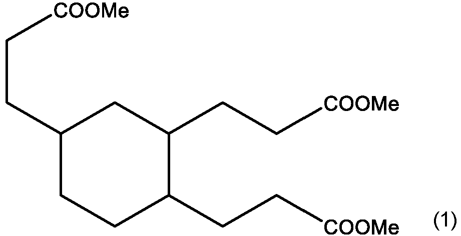 und mindestens einen 1,2-Dialkylcyclohexandicarbonsäureester, wobei die beiden Alkylgruppen jeweils 8 oder 9 Kohlenstoffatome aufweisen. Bevorzugte Ausführungsformen der vorliegenden Erfindung sind in den Unteransprüchen angegeben.

Der Trimethylester der 1,2,4-Cyclohexantripropionsäure, also die Substanz, die einen Teil der erfindungsgemäßen Weichmacherzusammensetzung darstellt, ist aktuell nicht kommerziell erhältlich. Ein Verfahren zur Herstellung dieser Ester wird aber beispielsweise in der EP 3 842 411 A1 beschrieben. Die Ester der 1,2,4-Cyclohexantripropionsäure sind somit allgemein zugänglich.

Neben den Trimethylestern der 1,2,4-Cyclohexantripropionsäure enthält die erfindungsgemäße Weichmacherzusammensetzung 1,2-Dialkylcyclohexandicarbonsäureester, wobei die beiden Alkylgruppen jeweils unabhängig voneinander 8 oder 9 Kohlenstoffatome aufweisen. Die entsprechenden Verbindungen können durch die nachfolgende Markush-Formel beschrieben werden: 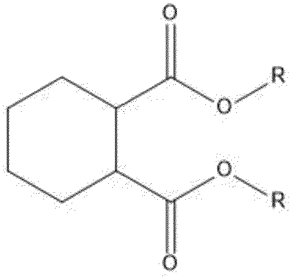 wobei die beiden Reste R jeweils unabhängig voneinander Alkylgruppen mit 8 oder 9 Kohlenstoffatomen sind.

Die beiden Alkylgruppen können dabei jeweils unabhängig voneinander aus der Gruppe, bestehend aus einer normalen Octylgruppe, einer Isooctylgruppe, einer 2-Ethylhexylgruppe, einer normalen Nonylgruppe und einer Isononylgruppe ausgewählt werden. Unter einer Isooctylgruppe wird im Sinne der vorliegenden Erfindung eine Mischung aus linearen und verzweigten primären C8-Alkoholen verstanden. Analog versteht man unter einer Isononylgruppe eine Mischung aus linearen und verzweigten C9-Alkoholen.

In einer bevorzugten Ausführungsform der vorliegenden Erfindung weisen die beiden Alkylgruppen des 1,2-Dialkylcyclohexandicarbonsäureesters jeweils die gleiche Anzahl an Kohlenstoffatomen auf. Das bedeutet, dass beide Alkylgruppen 8 oder beide Alkylgruppen 9 Kohlenstoffatome aufweisen, aber nicht, dass beide Alkylgruppen immer identisch sein müssen.

Es ist jedoch besonders bevorzugt, dass der 1,2-Dialkylcyclohexandicarbonsäureester in der erfindungsgemäßen Zusammensetzung 1,2-Di-isononyl-cyclohexandicarbonsäureester (DINCH) oder 1,2-Di-2-ethylhexyl-cyclohexandicarbonsäureester (DEHCH) ist.

Die Herstellung der erfindungsgemäßen 1,2-Dialkylcyclohexandicarbonsäureester kann über direkte Veresterung der 1,2-Cyclohexandicarbonsäure oder des entsprechenden Anhydrids mit einem Alkohol mit 8 oder 9 Kohlenstoffatomen erfolgen. Der Alkohol wird dabei vorzugsweise im Überschuss eingesetzt, d. h. in einer Menge von mehr als 2 Mol pro Mol Säurekomponente. Die direkte Veresterung wird weiterhin bevorzugt an einem sauren Katalysator durchgeführt, beispielsweise anorganische, organische oder Lewis-Säuren. Alternativ können die erfindungsgemäßen 1,2-Dialkylcyclohexandicarbonsäureester auch über eine Reaktionssequenz, die eine Diels-Alder-Reaktion aus Butadien und Maleinsäureanhydrid einschließt, hergestellt werden. Das so entstehende sog. Tetrahydrophthalsäureanhydrid wird anschließend mit Alkoholen verestert und die Doppelbindung hydriert.

Die Herstellung der erfindungsgemäßen 1,2-Dialkylcyclohexandicarbonsäureester kann auch über eine Umesterung erfolgen, wobei ein 1,2-Dialkylcyclohexandicarbonsäureester mit Alkylgruppen mit weniger als 8 Kohlenstoffatomen eingesetzt und mit einem Alkohol mit 8 oder 9 Kohlenstoffatomen umgeestert wird. Auch hier kann der Alkohol mit 8 oder 9 Kohlenstoffatomen im Überschuss eingesetzt werden.

Vorzugsweise erfolgt die Herstellung aber über die Veresterung von Phthalsäure, besonders bevorzugt mit Phthalsäureanhydrid mit einem Alkohol mit 8 oder 9 Kohlenstoffatomen oder durch Umesterung eines Phthalsäureesters, dessen Alkylgruppe weniger als 8 Kohlenstoffatome aufweist. Es entstehen dabei Dialkylphthalsäureester, deren Alkylgruppen 8 oder 9 Kohlenstoffatome aufweisen. Anschließend werden die so hergestellten Dialkylphthalsäureester kernhydriert, um die gewünschten 1,2-Dialkylcyclohexandicarbonsäureester zu erhalten. Verfahren zur Kernhydrierung sind in der Literatur beschrieben und dem Fachmann bekannt.

Die beiden Substanzen können in unterschiedlichen Mengen in der erfindungsgemäßen Weichmacherzusammensetzung vorhanden sein. Es sollte klar sein, dass die beiden Substanzen in einer Menge vorhanden sein müssen, bei der sie eine Wirkung entfalten, d. h. wo eine weichmachende Wirkung eintritt. In einer bevorzugten Ausführungsform der vorliegenden Erfindung liegen die Verbindung nach Formel (1) und der 1,2-Dialkylcyclohexandicarbonsäureester in einem Gewichtsverhältnis (Verbindung nach Formel (1) : 1,2-Dialkylcyclohexandicarbonsäureester) von 1 : 99 bis 99 : 1, vorzugsweise 10 : 90 bis 90 : 10, besonders bevorzugt 30 : 70 bis 70 : 30 in der Weichmacherzusammensetzung vor. In einer ganz besonders bevorzugten Ausführung der vorliegenden Erfindung liegen die Verbindung nach Formel (1) und der 1,2-Dialkylcyclohexandicarbonsäureester in einem Gewichtsverhältnis (Verbindung nach Formel (1) : 1,2-Dialkylcyclohexandicarbonsäureester) von 30 : 70 bis 5 : 95 in der Weichmacherzusammensetzung vor.

Die erfindungsgemäße Weichmacherzusammensetzung der vorliegenden Erfindung kann zusätzlich ein epoxidiertes Öl oder einen epoxidierten Alkylester einer Fettsäure enthalten. Epoxidierte Öle oder entsprechende Ester können die thermische Stabilität und die mechanischen Eigenschaften verbessern.

Das epoxidierte Öl kann aus der Gruppe bestehend aus epoxidiertem Sojabohnenöl, epoxidiertem Rizinusöl, epoxidiertem Leinöl, epoxidiertem Palmöl, epoxidiertem Tallöl und Mischungen davon ausgewählt werden. Bevorzugt werden epoxidiertes Sojabohnenöl oder epoxidiertes Leinöl in der erfindungsgemäßen Weichmacherzusammensetzung eingesetzt. Besonders bevorzugt ist epoxidiertes Sojabohnenöl, das auch unter dem Akronym ESBO bekannt ist.

Die epoxidierten Fettsäureester können durch Umesterung der o.g. epoxidierten Öle mit Alkoholen im Bereich von 1 bis 10 Kohlenstoffatomen, bevorzugt 4 bis 9 Kohlenstoffatomen, hergestellt werden. Alternativ kann erst das natürliche Öl umgeestert und die Doppelbindungen der Fettsäure anschließend epoxidiert werden.

Das epoxidierte Öl oder die entsprechenden epoxidierten Fettsäurealkylester können in einer Menge von 1 bis 150 Gewichtsteilen bezogen auf 100 Gewichtsteile der Gesamtsumme aus der Verbindung gemäß Formel (1) und dem 1,2-Dialkylcyclohexandicarbonsäureester eingesetzt werden. Das epoxidierte Öl oder die epoxidierten Fettsäureester können auch in Mengen von 2 bis 125 Gewichtsteilen, 5 bis 100 Gewichtsteilen, 10 bis 80 Gewichtsteilen oder 20 bis 70 Gewichtsteilen jeweils bezogen auf 100 Gewichtsteile der Gesamtsumme aus der Verbindung gemäß Formel (1) und dem 1,2-Dialkylcyclohexandicarbonsäureester in der Weichmacherzusammensetzung enthalten sein.

Die erfindungsgemäße Weichmacherzusammensetzung kann zusätzlich noch mindestens einen weiteren Weichmacher enthalten, der aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, epoxidierten Fettsäureestern, epoxidierten Pflanzenölen, epoxidierten acylierten Glyceriden, Furandicarboxylaten, Phosphaten, Succinaten, Sulfonamiden, Sulfonaten, Phthalaten, Terephthalaten, Isophthalaten, Trimellitaten, Cyclohexandicarboxylaten mit Ausnahme der bereits vorhandenen Ester und oligomeren oder polymeren Estern auf Basis von Adipin-, Bernstein- oder Sebacinsäure, ausgewählt wird.

In einer bevorzugten Ausführungsform der vorliegenden Erfindung enthält die Weichmacherzusammensetzung mindestens einen weiteren Weichmacher, der aus der Gruppe, bestehend aus Alkylbenzoaten, Alkylsulfonsäureestern des Phenols, Dialkyladipaten, Glycerinestern, C4-bis C6- Alkansäureester von Polyolen, acetylierten oder nicht acetylierten Citronensäuretrialkylestern, Glykoldibenzoaten, Trialkylestern der Trimellitsäure, Dialkylterephthalaten, Dialkylphthalaten, Dialkylisophthalaten, Estern der Furandicarbonsäure, Dialkanoylestern von Dianhydrohexitolen (z.B. Isosorbid), epoxidierten Fettsäurealkylestern, Polymerweichmachern, beispielsweise der Polyadipate, Dialkylestern der 1,3- oder 1,4-Cyclohexandicarbonsäure und Dialkylestern der 1,2-Cyclohexandicarbonsäure mit Ausnahme der bereits vorhandenen Ester mit 8 oder 9 Kohlenstoffatomen, ausgewählt wird.

In einer weiterhin bevorzugten Ausführungsform wird der mindestens eine weitere Weichmacher, der in der erfindungsgemäßen Weichmacherzusammensetzung enthalten sein kann, aus der Gruppe, bestehend aus C8- bis C13-Alkylbenzoaten, C4- bis C10-Dialkyladipaten, Pentaerythrit-tetravalerat, acetylierten oder nicht acetylierten Citronensäuretrialkylestern mit C4 bis C9-Alkylgruppen, C4- bis C10-Trialkyltrimellitaten, C4- bis C9-Dialkylterephthalaten, C4- bis C13-Dialkylphthalaten, insbesondere C9-bis C13-Dialkylphthalaten und C4- bis C10-Dialkylestern der 1,3- oder 1,4-Cyclohexandicarbonsäure, ausgewählt.

Besonders bevorzugt werden von den Citraten Butyl- oder Pentyl- oder höhere Citrate eingesetzt, die eine Acetylgruppe aufweisen können. Darunter fallen Tributylcitrat, Tripentylcitrat, Tri-n-hexylcitrat, Tri-2-ethylhexylcitrat, Triisononylcitrat, Acetyltributylcitrat und Acetyltripentylcitrat. Von den C4- bis C10-Dialkyladipaten sind Diethylhexyladipat (DEHA) und Diisononyladipat (DINA) bevorzugt. Von den C4- bis C9-Dialkylterephthalaten sind Dibutylterephthalat (DBT), Dipentylterephthalat (DPT) und Diethylhexylterephthalat (DEHT bzw. DOTP) bevorzugt. Von den C4- bis C10-Trialkyltrimellitaten sind Triethylhexyltrimellitat (TEHTM bzw. TOTM), und Triisononylterephthalat (TINTM) bevorzugt. Von den C4- bis C10-Dialkylestern der 1,4-Cyclohexandicarbonsäure sind der 1,4-Diethylhexylcyclohexandicarbonsäureester (1,4-DEHCH) und der 1,4-Diisononylcyclohexandicarbonsäureester (1,4-DINCH bzw. DINCD) bevorzugt.

Ein weiterer Gegenstand der vorliegenden Erfindung ist eine Kunststoffzusammensetzung, die einen Kunststoff und die Weichmacherzusammensetzung, umfassend die Verbindung der Formel (1) und mindestens einen 1,2-Dialkylcyclohexandicarbonsäureester, wobei die beiden Alkylgruppen jeweils 8 oder 9 Kohlenstoffatome aufweisen, enthält. Die Kunststoffzusammensetzung kann zudem das epoxidierte Öl und /oder ein epoxidierten Fettsäurealkylester und/oder mindestens einen zusätzlichen Weichmacher aus der oben genannten Liste enthalten.

Geeignete Kunststoffe sind polymere Substanzen, die vorzugsweise aus der Gruppe, bestehend aus PVC (Polyvinylchlorid), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi und Silikonen, ausgewählt werden.

In einer bevorzugten Ausführungsform ist der Kunststoff in der Weichmacherzusammensetzung ausgewählt aus der Gruppe bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat. Besonders bevorzugt ist hiervon PVC als Kunststoff in der erfindungsgemäßen Kunststoffzusammensetzung.

Die Menge an der erfindungsgemäßen Weichmacherzusammensetzung in der Kunststoffzusammensetzung beträgt vorzugsweise 5 bis 150 Gewichtsteile, bevorzugt 10 bis 120 Gewichtsteile, besonders bevorzugt 15 bis 110 Gewichtsteile und ganz besonders bevorzugt 20 bis 100 Gewichtsteile pro 100 Gewichtsteile des Kunststoffs.

Die Kunststoffzusammensetzung kann neben den erwähnten Inhaltsstoffen zusätzliche Additive enthalten. Beispiele für zusätzliche Additive sind rheologische Additive, mit denen die Viskosität der Kunststoffzusammensetzung verringert werden kann. Beispiele für bekannte rheologische Additive sind die unter den Handelsnamen VISCOBYK^{®}-5120, VISCOBYK^{®}-5130 und VISCOBYK^{®}-4041 erhältlichen Produkte. Die Additive können in einem Anteil von 1 bis 12, bevorzugt 2 bis 10 Gewichtsteilen pro 100 Gewichtsteilen PVC in der Kunststoffzusammensetzung enthalten sein.

Darüber hinaus kann die Kunststoffzusammensetzung einen oder mehrere Thermostabilisator(en) enthalten. Geeignete Thermostabilisatoren sind Bleisalze, Organozinnverbindungen, Barium/Zinkverbindungen, Cadmiumverbindungen oder Zinkverbindungen, Calcium/Zink-Stabilisatoren und organisch-basierte Stabilisatoren (sog. OBS). Bevorzugte Thermostabilisatoren sind Barium/Zinkverbindungen, Calcium/Zink-Stabilisatoren und organisch-basierte Stabilisatoren (sog. OBS). Der Anteil des oder der Stabilisatoren in der Kunststoffzusammensetzung beträgt vorzugsweise 1 bis 6 Gewichtsteile pro 100 Gewichtsteilen PVC.

Als weitere Additive können darüber hinaus auch Füllstoffe, Pigmente, Treibmittel und Gleitmittel in der Kunststoffzusammensetzung enthalten sein.

Die erfindungsgemäße Kunststoffzusammensetzung ist vorzugsweise Bestandteil eines Klebstoffs, einer Dichtungsmasse, einer Beschichtungsmasse, eines Lacks, einer Farbe, eines Plastisols, eines Dryblends, eines Schaums, eines Kunstleders, eines Fußbodenbelags, insbesondere dessen Deck- oder Schaumschicht, einer Dachbahn, eines Unterbodenschutzes, einer Gewebebeschichtung, eines Kabels, einer Drahtisolierung, eines Schlauchs, eines Extrusionsartikels, einer Folie, eines Gegenstands im Automobilinnenbereich, einer Tapete, einer Tinte, eines Spielzeugs, einer Kontaktfolie, einer Lebensmittelverpackung oder eines medizinischen Artikels, insbesondere eines Schlauchs oder eines Blutbeutels.

Gegenstand der vorliegenden Erfindung ist deshalb auch die Verwendung der Kunststoffzusammensetzung in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.

Die vorliegende Erfindung wird nachfolgend anhand von Beispielen illustriert. Die folgenden Beispiele sollen die Erfindung erläutern, ohne deren Anwendungsbreite, die sich aus der Beschreibung und den Patentansprüchen ergibt, einzuschränken.

### Beispiele

### Beispiel 1 - Herstellung von Cyclohexan-1,2,4-tripropionsäuretrimethylester (Me-Tc)

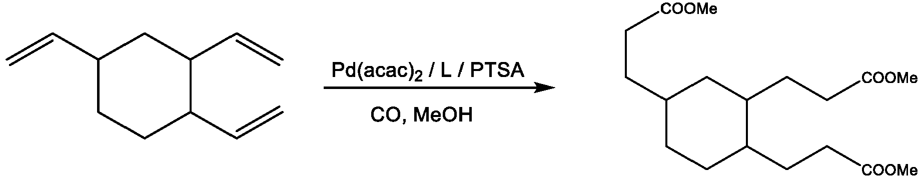

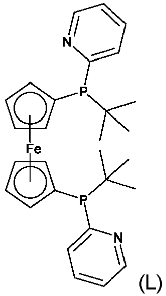

[Pd(acac)2] (15,2 mg, 0,1 mol%), der Katalysator L (103 mg, 0,4 mol%) und p-Toluolsulfonsäure (PTSA) (143 mg, 1,5 mol%) wurden unter einer Argonatmosphäre in einen 100-ml-Stahlautoklaven gegeben. Dann wurden MeOH (30 ml) und Trivinylcyclohexan (8,1 g, 50 mmol) mittels einer Spritze injiziert.

Der Autoklav wurde dreimal mit CO gespült und anschließend mit einem CO-Druck von 40 bar beaufschlagt. Die Reaktion erfolgte über 10 h bei 110 °C. Danach wurde der Autoklav auf Raumtemperatur abgekühlt und entspannt. Das gewünschte Produkt wurde durch Destillation (165 °C bei 10⁻³ bar) aufgereinigt und mit ¹H-, ¹³C-NMR und HR-MS charakterisiert (15,6 g, 91% Ausbeute).

### Beispiel 2 - Herstellung von Plastisolen

Es wurden PVC-Plastisole hergestellt, wie sie beispielsweise zur Fertigung von Deckstrichfilmen für Fußbodenbeläge verwendet werden. Die Angaben in der Plastisolrezeptur sind jeweils in Gewichtsteilen (phr). Die Rezeptur der Polymerzusammensetzung ist in den Tabellen 1 und 2 aufgelistet.

**Tabelle 1: Übersicht der hergestellten Plastisole (Teil 1).**

| Plastisol | 1* | 2 | 3 | 4* | 5* | 6 | 7 |
|---|---|---|---|---|---|---|---|
| | phr | phr | phr | phr | phr | phr | Phr |
| PVC (Vestolit^{®} P 1430 K 70 Ultra; Fa. Vestolit) | 100 | 100 | 100 | 100 | 100 | 100 | 100 |
| Me-Tc | 16,67 | | | 25 | 33,33 | | |
| Eastman Effusion^{™} (Dibutylterephthalat, Fa. Eastman) | | 16,67 | | | | 33,33 | |
| ELATUR^{®} DPT, (Diisopentylterephthalat, Fa. Evonik Oxeno & Co. KG) | | | 16,67 | | | | 33,33 |
| ELATUR^{®} CH (Cyclohexan-1,2-dicarbonsäurediisononyleste r, Fa. Evonik Oxeno GmbH & Co. KG) | 33,33 | 33,33 | 33,33 | 25 | 16,67 | 16,67 | 16,67 |
| epoxidiertes Sojabohnenöl als Co-Stabilisator (Edenol^{®} D81, Fa. Emery) | 3 | 3 | 3 | 3 | 3 | 3 | |
| Thermostabilisator auf Ca/Zn-Basis (Reagens MBL 197/9 PF) | 2 | 2 | 2 | 2 | 2 | 2 | |

| | | | | | | | |
|---|---|---|---|---|---|---|---|
| * = erfindungsgemäße Zusammensetzung phr = parts per hundred parts resin | | | | | | | |

**Tabelle 2: Übersicht der hergestellten Plastisole (Teil 2).**

| Plastisol | 8* | 9 | 10 | 11* | 12 | 13 |
|---|---|---|---|---|---|---|
| | phr | phr | phr | phr | phr | phr |
| PVC (Vestolit^{®} P 1430 K 70 Ultra; Fa. Vestolit) | 100 | 100 | 100 | 100 | 100 | 100 |
| Me-Tc | 16,67 | | | 33,33 | | |
| Eastman Effusion^{™} (Dibutylterephthalat, Fa. Eastman) | | 16,67 | | | 33,33 | |
| ELATUR^{®} DPT, (Diisopentylterephthalat, Fa. Evonik Oxeno & Co. KG) | | | 16,67 | | | 33,33 |
| EKFLEX 882 (Cyclohexan-1,2-dicarbonsäure-bis-(2-ethylhexylester, Fa. Elekeiroz/Brasilien) | 33,33 | 33,33 | 33,33 | 16,67 | 16,67 | 16,67 |
| epoxidiertes Sojabohnenöl als Co-Stabilisator (Edenol^{®} D81, Fa. Emery) | 3 | 3 | 3 | 3 | 3 | 3 |
| Thermostabilisator auf Ca/Zn-Basis (Reagens MBL 197/9 PF) | 2 | 2 | 2 | 2 | 2 | 2 |

| | | | | | | |
|---|---|---|---|---|---|---|
| * = erfindungsgemäße Zusammensetzung phr = parts per hundred parts resin | | | | | | |

Zuerst wurden die flüssigen und dann die pulverförmigen Bestandteile in einen PE-Becher eingewogen. Von Hand wurde die Mischung mit einem Spatel so eingerührt, dass kein unbenetztes Pulver mehr vorhanden war. Der verschlossene Becher wurde in die im Speedmixer befindliche Halterung gestellt und vermischt sowie entlüftet. Nach Beenden der Vermischung wurde die Temperatur des Plastisols mit Hilfe eines IR-Thermometers gemessen. Danach wurde das Plastisol sofort für weitere Untersuchungen in einem Klimaschrank bei 25,0 °C temperiert.

### Beispiel 3 - Gelierverhalten der Plastisole

Die Untersuchung des Gelierverhaltens der Plastisole aus Beispiel 2 wurde mittels Physica MCR 101 im Oszillationsmodus mit einem Platte-Platte Messsystem (PP25), welches schubspannungsgesteuert betrieben wurde, vorgenommen. Eine zusätzliche Temperierhaube wurde an das Gerät angeschlossen, um eine homogene Wärmeverteilung und eine gleichmäßige Probentemperatur zu erreichen.

Folgende Parameter wurden eingestellt:

| | |
|---|---|
| Modus: | Temperatur-Gradient |
| Start-Temperatur: | 25 °C |
| End-Temperatur: | 180 °C |
| Heiz/Kühlrate: | 5 °C/min |
| Oszillations-Frequenz: | 4 bis 0,1 Hz Rampe logarithmisch |
| Kreisfrequenz Omega: | 10 s⁻¹ |
| Anzahl Messpunkte: | 63 |
| Messpunktdauer: | 0,5 min |
| Automatische Spaltnachführung F: | 0 N |
| Konstante Messpunktdauer Spaltweite | 0,5 mm |

### Durchführung der Messung

Auf die untere Messsystemplatte wurden mit dem Spatel einige Gramm der zu messenden Paste luftblasenfrei aufgetragen. Dabei wurde darauf geachtet, dass nach dem Zusammenfahren des Messsystems etwas Paste gleichmäßig aus dem Messsystem herausquellen konnte (nicht mehr als 6 mm rundum). Der Überschuss wurde mittels Spatel entfernt. Anschließend wurde die Temperierhaube über der Probe positioniert und die Messung gestartet. Bestimmt wurde die komplexe Viskosität der Paste nach 24 Stunden (Lagerung der Paste bei 25 °C in einem Temperierschrank der Fa. Memmert) in Abhängigkeit von der Temperatur.

Als Maß für die Gelierung wurde ein deutlicher Anstieg der komplexen Viskosität betrachtet. Als Vergleichswert wurde daher die Temperatur bei Erreichen einer Pastenviskosität von 1000 Pa*s verwendet. Die erhaltenen Ergebnisse sind in Tabelle 3 aufgeführt.

**Tabelle 3: Gelierung der Plastisole nach 24 Stunden, Temperatur in °C bei Erreichen einer Pastenviskosität von 1000 Pa*s**

| Rezeptur | Weichmacherzusammensetzung nach Tabelle 1 und 2 | Geliertemperatur [°C] |
|---|---|---|
| 1* | DINCH : Me-Tc (2:1) | 72,1 |
| 2 | DINCH : DBT (2:1) | 73,8 |
| 3 | DINCH : DPT (2:1) | 77,9 |
| 4* | DINCH : Me-Tc (1:1) | 68,0 |
| 5* | DINCH : Me-Tc (1:2) | 65,2 |
| 6 | DINCH : DBT (1:2) | 65,9 |
| 7 | DINCH : DPT (1:2) | 72,3 |
| 8* | 1,2-DEHCH : Me-Tc (2:1) | 69,9 |
| 9 | 1,2-DEHCH : DBT (2:1) | 71,6 |
| 10 | 1,2-DEHCH : DPT (2:1) | 74,6 |
| 11* | 1,2-DEHCH : Me-Tc (1:2) | 64,9 |
| 12 | 1,2-DEHCH : DBT (1:2) | 65,1 |
| 13 | 1,2-DEHCH : DPT (1:2) | 70,1 |

| | | |
|---|---|---|
| * Versuch mit erfindungsgemäßer Weichmacherzusammensetzung | | |

Im Vergleich mit anderen Schnellgelierern sorgt der Einsatz des Cyclohexan-1,2,4-tripropionsäure-trimethylesters (Me-Tc) in einer Weichmacherzusammensetzung für eine deutliche Reduktion der Geliertemperatur. Wird mehr Me-Tc eingesetzt, kann die Geliertemperatur weiter gesenkt werden.

### Beispiel 4 - Herstellung von Folien

Die in Beispiel 2 hergestellten Plastisole wurden jeweils zu 1 mm dicken Folien verarbeitet. Dazu wurde zunächst Hochglanztrennpapier (Fa. Sappi, Italien) auf eine Größe von 30 x 44 cm zugeschnitten und im Spannrahmen der Streicheinrichtung LTSV für den Mathis-Ofen eingelegt. Danach wurde der Spannrahmen auf den Führungsrahmen aufgelegt, der Mathis-Ofen (Typ LTF) auf 200 °C eingestellt und der Rahmen nach Erreichen dieser Temperatur 15 Sekunden vorgewärmt. Danach wurde die Rakel in die Einspannvorrichtung gelegt und der Rakelspalt über Vorversuche so eingestellt, dass die Foliendicke nach Abschluss der Gelierung 1 mm (+/- 0,05 mm) betrug. Auf den vorderen Rand des Papiers wurde ein Klebestreifen angebracht, um überschüssiges Plastisol aufzufangen. Danach wurde das Plastisol vor der Rakel aufgetragen und diese durch Ziehen des Führungsrahmens mit der Rakel über das eingespannte Trennpapier verstrichen (3 m/min Geschwindigkeit). Danach wurde die Rakel herausgenommen und der Klebestreifen mit dem überschüssigen Plastisol abgenommen. Anschließend wurde der Spannrahmen in den Ofen eingefahren. Nach der Gelierung (2 Minuten bei 200 °C) wurde der Rahmen wieder aus dem Ofen herausgefahren und nach Abkühlen die Folie von dem Papier abgezogen.

### Beispiel 5 - Bestimmung des Masseverlusts (Aktivkohle-Verfahren)

In Anlehnung an DIN EN ISO 176 (Verfahren B) wurden aus den hergestellten PVC- Folien jeweils drei kreisrunde Scheiben mit einem Durchmesser von 5 cm ausgestanzt und zunächst für 16 Stunden im Exsikkator bei Raumtemperatur konditioniert. Auf der Analysenwaage wurden die Proben-Kreise ausgewogen, in einen Drahtkorb gelegt, welcher mit einer Klammer verschlossen wurde. Die Drahtkörbe wurden anschließend in eine mit Aktivkohle gefüllte Weißblechdose so platziert, dass sich zwischen den einzelnen Körben bzw. Korb und Deckel (gelocht) bzw. Boden jeweils etwa 130 ml Aktivkohle befanden.

Die befüllten Weißblechdosen wurden im vortemperierten Heizschrank (120 °C) so platziert, dass der Lüfter im Schrank nicht zugestellt wurde und die Dosen sich nicht berührten. Luftwechsel und Abluftklappe wurden auf jeweils 10 % eingestellt. Nach 72 Stunden wurden die Dosen wieder aus dem Schrank entnommen, abgekühlt, danach die einzelnen Proben den Körben entnommen, im Exsikkator nochmals 16 Stunden konditioniert und danach auf der Analysenwaage zurückgewogen. Die erhaltene Massedifferenz entsteht durch den Verlust an Weichmacher. Von den jeweils drei Massedifferenzen wurde der Mittelwert gebildet und prozentual der Verlust an Weichmacher errechnet. In Tabelle 4 sind die Ergebnisse aufgeführt.

**Tabelle 4: Massenverlust der Prüfkörper**

| Rezeptur | Weichmacherzusammensetzung nach Tabelle 1 und 2 | Massenverlust nach 3 Tagen in % |
|---|---|---|
| 1* | DINCH : Me-Tc (2:1) | 5,9 |
| 2 | DINCH : DBT (2:1) | 12,8 |
| 3 | DINCH : DPT (2:1) | 9,4 |
| 4* | DINCH : Me-Tc (1:1) | 6,1 |
| 5* | DINCH : Me-Tc (1:2) | 6,5 |
| 6 | DINCH : DBT (1:2) | 21,0 |
| 7 | DINCH : DPT (1:2) | 14,9 |
| 8* | 1,2-DEHCH : Me-Tc (2:1) | 10,5 |
| 9 | 1,2-DEHCH : DBT (2:1) | 17,7 |
| 10 | 1,2-DEHCH : DPT (2:1) | 14,3 |
| 11* | 1,2-DEHCH : Me-Tc (1:2) | 9,4 |
| 12 | 1,2-DEHCH : DBT (1:2) | 23,7 |
| 13 | 1,2-DEHCH : DPT (1:2) | 17,6 |

Der Massenverlust von Folien mit den erfindungsgemäßen Weichmacherzusammensetzungen sind geringer als der von Folien, die mit Weichmacherzusammensetzungen, die andere Schnellgelierer enthalten, hergestellt worden sind. Vergleicht man die Masseverluste der Gemische 1, 4 und 5 sowie 8 und 11 so erkennt man überraschenderweise einen nur geringen Anstieg der Masseverluste trotz deutlich steigender Anteile des Schnellgelierers Me-Tc.

### Beispiel 6 - Bestimmung der Migrationseigenschaften

Der Test zur Bestimmung der Migrationseigenschaften eines Prüfkörpers, der die zu prüfenden Weichmacherzusammensetzungen enthält, erlaubt es, Rückschlüsse darauf zu ziehen, wie sich Weichmacher-haltige Polymerkörper in der Anwendung bei direktem Kontakt mit anderen Materialien - wie beispielsweise bei Kontakt zwischen einer weichgemachten PVC-Schicht mit einer Polystyrol-Schicht - verhalten (so genannte "Multi-Layer Systeme"). Geht beispielsweise innerhalb eines Artikels von einem weichgemachten Polymer-haltigen Bauteil Weichmacher zu einem nicht-weichgemachten Polymer-haltigen Bauteil über, kann dies zur unerwünschten Versprödung des weichgemachten Bauteils und Aufweichung des nicht-weichgemachten Bauteils führen, was zu Undichtigkeiten, nachlassender Stabilität und verringerter Gebrauchszeit des Artikels führen kann. Dementsprechend ist eine geringe Migrationsneigung eine wichtige Eigenschaft für einen in zahlreichen Anwendungen einsetzbaren Weichmacher oder eine dementsprechende Komponente einer Weichmacherzusammensetzung.

Die Migrationsneigung der oben genannten Weichmacherzusammensetzungen wurde wie folgt bestimmt:
Die nach Beispiel 4 hergestellten, 1 mm dicken Folien wurden in Prüflinge von 100 mm x 100 mm geschnitten und für 24 Stunden bei 23 °C gelagert, bevor ihre Masse mit einer Analysenwaage ermittelt wurde.

In Anlehnung an die Vorschrift DIN EN ISO 177 (veröffentlicht 1999) wurden die Prüflinge zwischen zwei Kontaktschichten (hochschlagfestes Polystyrol (HIPS), Hersteller VINK Kunststoffe; 100 x 100 x 2 mm Platten), welche bis auf eine Stärke von 2 mm dieselben Dimensionen wie die Prüflinge aufwiesen, platziert und jeweils zwei der auf diese Weise entstehenden Schichtkonstruktionen übereinander gestapelt. Diese Stapel umfassend jeweils Prüflinge desselben Weichmachers wurden mit einem 2 kg Gewicht beschwert für 28 Tage in einem Ofen (70 (+/- 1) °C) gelagert.

Nach 14 Tagen und nach 28 Tagen wurde der Gewichtsverlust an jedem Prüfling bestimmt (Prozent Gewichtsverlust basierend auf dem Originalgewicht). Nach der ersten Messung wurden die Stapel wieder wie zuvor zusammengesetzt. Die Tabelle 5 zeigt die gemittelten Werte des Gewichtsverlustes der Prüfkörper.

**Tabelle 5: Migration in hochschlagfestes Polystyrol (HIPS) nach 14 bzw. 28 Tagen**

| Rezeptur | Weichmacherzusammensetzung nach Tabelle 1 und 2 | Gewichtsdifferenz nach 14 Tagen in % | Gewichtsdifferenz nach 28 Tagen in % |
|---|---|---|---|
| 1* | DINCH : Me-Tc (2:1) | 13,6 | 16,0 |
| 2 | DINCH : DBT (2:1) | 15,2 | 17,8 |
| 3 | DINCH : DPT (2:1) | 14,6 | 17,3 |
| 4* | DINCH : Me-Tc (1:1) | 11,6 | 14,8 |
| 5* | DINCH : Me-Tc (1:2) | 10,8 | 13,5 |
| 6 | DINCH : DBT (1:2) | 15,1 | 18,2 |
| 7 | DINCH : DPT (1:2) | 14,7 | 17,5 |
| 8* | 1,2-DEHCH : Me-Tc (2:1) | 12,0 | 15,2 |
| 9 | 1,2-DEHCH : DBT (2:1) | 14,6 | 17,5 |
| 10 | 1,2-DEHCH : DPT (2:1) | 14,3 | 17,0 |
| 11* | 1,2-DEHCH : Me-Tc (1:2) | 10,9 | 13,3 |
| 12 | 1,2-DEHCH : DBT (1:2) | 15,2 | 17,9 |
| 13 | 1,2-DEHCH : DPT (1:2) | 13,0 | 16,1 |

Es ist zu erkennen, dass die erfindungsgemäßen Weichmacherzusammensetzungen eine niedrigere Migrationsneigung aufweisen als Weichmacherzusammensetzungen, die andere Schnellgelierer enthalten.

## Patentansprüche

1. Weichmacherzusammensetzung enthaltend eine Verbindung der Formel (1) 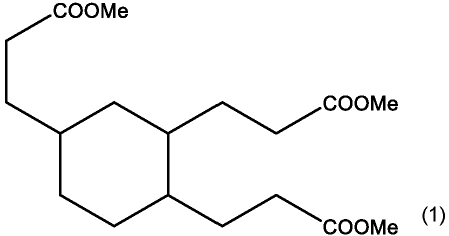 und mindestens einen 1,2-Dialkylcyclohexandicarbonsäureester, wobei die beiden Alkylgruppen jeweils 8 oder 9 Kohlenstoffatome aufweisen.

2. Weichmacherzusammensetzung nach Anspruch 1, wobei die beiden Alkylgruppen jeweils unabhängig voneinander aus der Gruppe, bestehend aus einer normalen Octylgruppe, einer Isooctylgruppe, einer 2-Ethylhexylgruppe, einer normalen Nonylgruppe und einer Isononylgruppe ausgewählt werden.

3. Weichmacherzusammensetzung nach Anspruch 1 oder 2, wobei die beiden Alkylgruppen die gleiche Anzahl an Kohlenstoffatomen aufweisen.

4. Weichmacherzusammensetzung nach einem der vorhergehenden Ansprüche, wobei der 1,2-Dialkylcyclohexandicarbonsäureester der 1,2-Di-isononyl-cyclohexandicarbonsäureester (DINCH) oder der 1,2-Di-2-ethylhexylcyclohexandicarbonsäureester (DEHCH) ist.

5. Weichmacherzusammensetzung nach einem der vorhergehenden Ansprüche, wobei die Verbindung nach Formel (1) und der 1,2-Dialkylcyclohexandicarbonsäureester in einem Gewichtsverhältnis (Verbindung nach Formel (1) : 1,2-Dialkylcyclohexandicarbonsäureester) von 1 : 99 bis 99 : 1, vorzugsweise 10 : 90 bis 90 : 10, besonders bevorzugt 30 : 70 bis 70 : 30 in der Weichmacherzusammensetzung vorhanden sind.

6. Weichmacherzusammensetzung nach einem der vorhergehenden Ansprüche, wobei die Weichmacherzusammensetzung zusätzlich ein epoxidiertes Öl oder einen epoxidierten Alkylester einer Fettsäure enthält.

7. Weichmacherzusammensetzung nach Anspruch 6, wobei das epoxidierte Öl aus der Gruppe bestehend aus epoxidiertem Sojabohnenöl, epoxidiertem Rizinusöl, epoxidiertem Leinöl, epoxidiertem Palmöl, epoxidiertem Stearat, epoxidiertem Oleat, epoxidiertem Tallöl, epoxidiertem Linoleat und Mischungen davon ausgewählt wird.

8. Weichmacherzusammensetzung nach Anspruch 7, wobei das epoxidierte Öl epoxidiertes Sojabohnenöl oder epoxidiertes Leinöl, vorzugsweise epoxidiertes Sojabohnenöl ist.

9. Weichmacherzusammensetzung nach einem der Ansprüche 6 bis 8, wobei das epoxidierte Öl in einer Menge von 1 bis 150 Gewichtsteilen bezogen auf 100 Gewichtsteile der Gesamtsumme aus der Verbindung gemäß Formel (1) und dem 1,2-Dialkylcyclohexandicarbonsäureester in der Weichmacherzusammensetzung vorhanden ist.

10. Weichmacherzusammensetzung nach einem der vorherigen Ansprüche, wobei die Zusammensetzung zusätzlich noch einen weiteren Weichmacher aus der Gruppe, bestehend aus Adipaten, Benzoaten, beispielsweise Monobenzoaten oder Glycoldibenzoaten, chlorierten Kohlenwasserstoffen (sog. Chlorparaffinen), Citraten, epoxidierten Fettsäureestern, epoxidierten Pflanzenölen, epoxidierten acylierten Glyceriden, Furandicarboxylaten, Phosphaten, Succinaten, Sulfonamiden, Sulfonaten, Terephthalaten, Isophthalaten, Trimellitaten, Cyclohexandicarboxylaten und oligomeren oder polymeren Estern auf Basis von Adipin-, Bernstein- oder Sebacinsäure, ausgewählt werden.

11. Kunststoffzusammensetzung, umfassend die Weichmacherzusammensetzung nach einem der Ansprüche 1 bis 10 und einen Kunststoff.

12. Kunststoffzusammensetzung nach Anspruch 11, wobei der Anteil der Weichmacherzusammensetzung 5 bis 150 Gewichtsteile pro 100 Gewichtsteile Kunststoff beträgt.

13. Kunststoffzusammensetzung nach Anspruch 11 oder 12, wobei der Kunststoff aus der Gruppe, bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat, ausgewählt wird.

14. Kunststoffzusammensetzung nach Anspruch 13, wobei der Kunststoff Polyvinylchlorid (PVC) ist.

15. Verwendung der Kunststoffzusammensetzung nach einem der Ansprüche 11 bis 14 in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.

## Claims

1. Plasticizer composition comprising a compound of the formula (1) 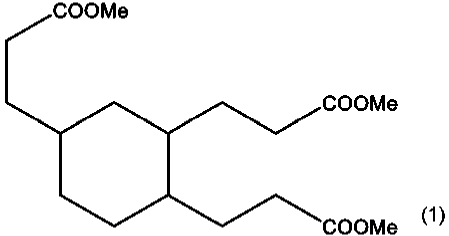 and at least one dialkyl cyclohexane-1,2-dicarboxylate, where the two alkyl groups each have 8 or 9 carbon atoms.

2. Plasticizer composition according to Claim 1, wherein the two alkyl groups are each independently selected from the group consisting of a normal octyl group, an isooctyl group, a 2-ethylhexyl group, a normal nonyl group and an isononyl group.

3. Plasticizer composition according to Claim 1 or 2, wherein the two alkyl groups have the same number of carbon atoms.

4. Plasticizer composition according to any of the preceding claims, wherein the dialkyl cyclohexane-1,2-dicarboxylate is diisononyl cyclohexane-1,2-dicarboxylate (DINCH) or di-2-ethylhexyl cyclohexane-1,2-dicarboxylate (DEHCH).

5. Plasticizer composition according to any of the preceding claims, wherein the compound of formula (1) and the dialkyl cyclohexane-1,2-dicarboxylate are present in the plasticizer composition in a weight ratio (compound of formula (1): dialkyl cyclohexane-1,2-dicarboxylate) of 1: 99 to 99: 1, preferably 10: 90 to 90: 10, more preferably 30: 70 to 70: 30.

6. Plasticizer composition according to any of the preceding claims, wherein the plasticizer composition additionally comprises an epoxidized oil or an epoxidized alkyl ester of a fatty acid.

7. Plasticizer composition according to Claim 6, wherein the epoxidized oil is selected from the group consisting of epoxidized soybean oil, epoxidized castor oil, epoxidized linseed oil, epoxidized palm oil, epoxidized stearate, epoxidized oleate, epoxidized tall oil, epoxidized linoleate and mixtures thereof.

8. Plasticizer composition according to Claim 7, wherein the epoxidized oil is epoxidized soybean oil or epoxidized linseed oil, preferably epoxidized soybean oil.

9. Plasticizer composition according to any of Claims 6 to 8, wherein the epoxidized oil is present in the plasticizer composition in an amount of 1 to 150 parts by weight based on 100 parts by weight of the sum total of the compound of formula (1) and the dialkyl cyclohexane-1,2-dicarboxylate.

10. Plasticizer composition according to any of the preceding claims, wherein the composition additionally a further plasticizer from the group consisting of adipates, benzoates, for example monobenzoates or glycol dibenzoates, chlorinated hydrocarbons (called chloroparaffins), citrates, epoxidized fatty acid esters, epoxidized vegetable oils, epoxidized acylated glycerides, furandicarboxylates, phosphates, succinates, sulfonamides, sulfonates, terephthalates, isophthalates, trimellitates, cyclohexanedicarboxylates and oligomeric or polymeric esters based on adipic acid, succinic acid or sebacic acid are selected.

11. Plastics composition comprising the plasticizer composition according to any of Claims 1 to 10 and a plastic.

12. Plastics composition according to Claim 11, wherein the proportion of the plasticizer composition is 5 to 150 parts by weight per 100 parts by weight of plastic.

13. Plastics composition according to Claim 11 or 12, wherein the plastic is selected from the group consisting of polyvinylchloride (PVC), polyalkylmethacrylate (PAMA), polyvinylbutyral (PVB), polyurethane, polysulfides, polylactic acid (PLA), polyhydroxybutyral (PHB), nitrocellulose and copolymers of vinyl chloride with vinyl acetate or with butyl acrylate.

14. Plastics composition according to Claim 13, wherein the plastic is polyvinylchloride (PVC).

15. Use of the plastics composition according to any of Claims 11 to 14 in adhesives, sealing compounds, coating compounds, varnishes, paints, plastisols, foams, imitation leathers, floor coverings, especially the top layer and foam layer, roofing membranes, underbody protection, fabric coatings, cables, wire insulation, hoses, extruded articles, films, in the automotive interior sector, in wallpaper, inks, toys, contact sheets, food packaging or medical articles, especially in tubes or blood bags.

## Revendications

1. Composition plastifiante, contenant un composé de formule (1) 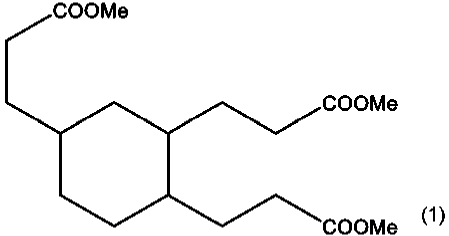 et au moins un ester 1,2-dialkylique de l'acide cyclohexanedicarboxylique, les deux groupes alkyle présentant à chaque fois 8 ou 9 atomes de carbone.

2. Composition plastifiante selon la revendication 1, les deux groupes alkyle étant choisis, à chaque fois indépendamment l'un de l'autre, dans le groupe constitué par un groupe octyle normal, un groupe isooctyle, un groupe 2-éthylhexyle, un groupe nonyle normal et un groupe isononyle.

3. Composition plastifiante selon la revendication 1 ou 2, les deux groupes alkyle présentant le même nombre d'atomes de carbone.

4. Composition plastifiante selon l'une quelconque des revendications précédentes, l'ester 1,2-dialkylique de l'acide cyclohexanedicarboxylique étant l'ester 1,2-di-isononylique de l'acide cyclohexanedicarboxylique (DINCH) ou l'ester 1,2-di-2-éthylhexylique de l'acide cyclohexanedicarboxylique (DEHCH).

5. Composition plastifiante selon l'une quelconque des revendications précédentes, dans laquelle le composé selon la formule (1) et l'ester 1,2-dialkylique d'acide cyclohexanedicarboxylique sont présents selon un rapport pondéral (composé selon la formule (1) : ester 1,2-dialkylique d'acide cyclohexanedicarboxylique) de 1 : 99 à 99 : 1, de préférence de 10 : 90 à 90 : 10, de manière particulièrement préférée de 30 : 70 à 70 : 30 dans la composition plastifiante.

6. Composition plastifiante selon l'une quelconque des revendications précédentes, la composition plastifiante contenant en plus une huile époxydée ou un ester alkylique époxydé d'un acide gras.

7. Composition plastifiante selon la revendication 6, l'huile époxydée étant choisie dans le groupe constitué par l'huile de germes de soja époxydée, l'huile de ricin époxydée, l'huile de lin époxydée, l'huile de palme époxydée, le stéarate époxydé, l'oléate époxydé, l'huile de tall époxydée, le linoléate époxydé et leurs mélanges.

8. Composition plastifiante selon la revendication 7, l'huile époxydée étant l'huile de germes de soja époxydée ou l'huile de lin époxydée, de préférence l'huile de germes de soja époxydée.

9. Composition plastifiante selon l'une quelconque des revendications 6 à 8, l'huile époxydée étant présente en une quantité de 1 à 150 parties en poids par rapport à 100 parties en poids de la quantité totale du composé selon la formule (1) et de l'ester 1,2-dialkylique de l'acide cyclohexanedicarboxylique dans la composition plastifiante.

10. Composition plastifiante selon l'une quelconque des revendications précédentes, la composition, et en plus encore un autre plastifiant du groupe constitué par les adipates, les benzoates, par exemple les monobenzoates ou les dibenzoates de glycol, les hydrocarbures chlorés (appelés chloroparaffines), les citrates, les esters d'acide gras époxydés, les huiles végétales époxydées, les glycérides acylés époxydés, les furannedicarboxylates, les phosphates, les succinates, les sulfonamides, les sulfonates, les téréphtalates, les isophtalates, les trimellitates, les cyclohexanedicarboxylates et les esters oligomères ou polymères à base d'acide adipique, succinique ou sébacique étant choisis.

11. Composition de matériau synthétique comprenant la composition plastifiante selon l'une quelconque des revendications 1 à 10 et un matériau synthétique.

12. Composition de matériau synthétique selon la revendication 11, la proportion de composition plastifiante représentant 5 à 150 parties en poids par 100 parties en poids de matériau synthétique.

13. Composition de matériau synthétique selon la revendication 11 ou 12, le matériau synthétique étant choisi dans le groupe constitué par le poly(chlorure de vinyle) (PVC), le poly(méthacrylate d'alkyle) (PAMA), le polyvinylbutyral (PVB), le polyuréthane, le polysulfure, le poly(acide lactique) (PLA), le polyhydroxybutyral (PHB), la nitrocellulose et les copolymères du chlorure de vinyle avec de l'acétate de vinyle ou de l'acrylate de butyle.

14. Composition de matériau synthétique selon la revendication 13, le matériau synthétique étant le poly(chlorure de vinyle) (PVC).

15. Utilisation de la composition de matériau synthétique selon l'une quelconque des revendications 11 à 14 dans les adhésifs, les masses d'étanchéité, les masses de revêtement, les vernis, les peintures, les plastisols, les mousses, le cuir synthétique, les revêtements de sol, en particulier la couche de recouvrement et la couche de mousse, les membranes de toiture, la protection de bas de caisse, les revêtements de tissu, les câbles, les isolations de fil, les flexibles, les articles extrudés, les feuilles, à l'intérieur de véhicules, dans les tapis, les encres, les jouets, les films de contact, les emballages alimentaires ou les articles médicaux, en particulier dans les flexibles ou les poches à sang.
